# EUROPEAN PATENT APPLICATION

(11) **EP 4 008 235 A1**
(43) Date of publication of application: **08.06.2022**
(21) Application number: 20879443.8
(22) Date of filing: 05.10.2020
(51) Int. Cl.: A61B 1/045, G06T 5/00

(54) **IMAGE PROCESSING DEVICE, IMAGE PROCESSING METHOD, AND ENDOSCOPE SYSTEM**

(30) Priority: 21.10.2019 JP 2019192049
(71) Applicant: Sony Group Corporation, Tokyo 108-0075 (JP)
(72) Inventor: FUKAZAWA, Takanori, Tokyo 108-0075 (JP); TAKAHASHI, Minori, Tokyo 108-0075 (JP)
(74) Representative: D Young & Co LLP
(86) International application number: PCT/JP2020/037727
(87) International publication number: WO 2021/079723

(57) **Abstract**

To provide an image processing apparatus, an image processing method, and an endoscope system by which deep tissues and superficial blood vessels enhanced with improved visibility can be displayed to a surgeon in real time. An image processing apparatus according to an embodiment of the present technology includes an enhancement processing unit that performs enhancement processing on low-frequency components that are a range lower than a predetermined spatial frequency in an input image, performs enhancement processing on high-frequency components that are a range higher than the low-frequency components in the input image, and outputs the input image having the low-frequency components and the high-frequency components each subjected to enhancement processing.

## Description

### Technical Field

The present technology relates to an image processing apparatus, an image processing method, and an endoscope system for medical purposes.

### Background Art

In surgery, identifying positions of deep tissues such as blood vessels and lymph nodes covered with biological membranes and fat and performing suitable treatment are important for reducing the rate of complications and the surgery time and greatly contribute to an improvement in surgery safety.

As to medical images in recent years, the visibility of superficial tissue structures has been improved because of the increase in resolution. However, absorption and scattering blurring of reflected light due to the influence of biological membranes and fat lower the contrast of deep tissues, and it is thus desirable to further improve the visibility.

For example, Patent Literature 1 has disclosed a method of analyzing a superficial structure of a biological tissue to distinguish mucosal membranes and non-mucosal parts and performing display in which superficial blood vessels are enhanced. Patent Literature 2 has disclosed a method of applying edge enhancement to a medical image and performing display while preventing excessive enhancement in accordance with edge strength. In Patent Literatures 1 and 2, the visibility of deep tissues is not improved while the visibility of superficial blood vessels is improved.

It is because both methods mainly enhance high-frequency components of an image, such as blood vessels and edges. Since deep tissues are covered with biological membranes, fat, and the like, scattering blurring is greater and more low-frequency components are included as compared to superficial tissues. Therefore, low-frequency enhancement is required for improving the visibility of deep blood vessels.

### Citation List

### Patent Literature

Patent Literature 1: WO 2012/147505
Patent Literature 2: Japanese Patent Application Laid-open No. HEI 9-62836

### Disclosure of Invention

### Technical Problem

One of low-frequency enhancement processing means is unsharp masking. In the unsharp masking, an enhanced image is generated by determining a difference image between a smoothed image obtained by performing smoothing processing on an input image and the input image and combining the difference image with the input image.

During the smoothing processing phase of this method, the smoothed image in which the input image is more strongly blurred can be generated if the filter size is increased, and the difference image taking the difference between the input image and the smoothed image contains more low-frequency components. Therefore, stronger low-frequency enhancement can be achieved consequently.

However, the enhancement processing using the unsharp masking is processing of uniformly enhancing a band above a certain frequency band, and therefore it is difficult to enhance deep tissues and superficial blood vessels of a medical image at the same time, which imposes limitations on the degree of freedom in enhancement.

In view of the above-mentioned circumstances, it is an object of the present technology to provide an image processing apparatus, an image processing method, and an endoscope system by which deep tissues and superficial blood vessels with improved visibility can be displayed to a surgeon.

### Solution to Problem

An image processing apparatus according to an embodiment of the present technology includes an enhancement processing unit.

The enhancement processing unit performs enhancement processing on low-frequency components that are a range lower than a predetermined spatial frequency in an input image, performs enhancement processing on high-frequency components that are a range higher than the low-frequency components in the input image, and outputs the input image having the low-frequency components and the high-frequency components each subjected to enhancement processing.

The image processing apparatus can perform enhancement processing on each of the low-frequency component and the high-frequency component of the input image, output and display the image with improved visibility, and support the operation of a user who uses the image.

The enhancement processing unit may include a low-frequency enhancement processing unit that performs enhancement processing with respect to the low-frequency components of the input image, and the low-frequency enhancement processing unit may smooth the input image and obtain a difference image on the basis of a difference between the input image after smoothing and the input image before smoothing.

Accordingly, the low-frequency components of the input image can be enhanced and displayed.

The low-frequency enhancement processing unit may perform reduction processing on resolution of the input image at a predetermined reduction rate before the input image is smoothed.

Accordingly, reducing the input image in advance can reduce the amount of arithmetic operation of the smoothing filter and can perform smoothing at higher speed.

The low-frequency enhancement processing unit may increase resolution of the difference image at an increase rate corresponding to the predetermined reduction rate after the input image is smoothed.

Accordingly, the output image can be displayed with the original size before reduction.

The low-frequency enhancement processing unit may output a low-frequency-enhanced image obtained by multiplying an image having the resolution increased by a predetermined coefficient and combining the multiplied image with the input image.

Accordingly, the level of the low-frequency enhancement can be adjusted.

The enhancement processing unit may combine a low-frequency-enhanced image, which is an image having the low-frequency components subjected to enhancement processing, with the input image and perform enhancement processing on the high-frequency components of the input image combined with the low-frequency-enhanced image.

Accordingly, processing that does not enhance high-frequency noise components contained in the input image can be performed.

The enhancement processing unit may combine a low-frequency-enhanced image, which is an image having the low-frequency components subjected to enhancement processing, and a high-frequency enhanced image, which is an image with the high-frequency components subjected to enhancement processing, with the input image.

Accordingly, an image (moving image) with improved processing speed can be displayed to a user of the image processing apparatus.

The low-frequency enhancement processing unit may include a separation processing unit that separates the input image into a luminance component image and a chrominance component image, and a gain adjustment unit that selects, from the luminance component image and the chrominance component image, pixels to be enhanced and pixels not to be enhanced, and adjusts gains to be multiplied with respect to the pixels to be enhanced and the pixels not to be enhanced.

Accordingly, excessive enhancement can be suppressed by performing enhancement control not to enhance portions where the luminance component is dark and bright.

The input image may be a medical image.

Accordingly, deep tissues and superficial blood vessels with improved visibility can be displayed to a surgeon for support.

The input image may include at least one of a white light image illuminated in white, a narrow band image illuminated with narrow-band light, or a fluorescent image illuminated with excitation light.

Accordingly, superficial blurring can be removed while increasing the contrast of deep tissues.

In a case where two or more kinds of input images are input, the enhancement processing unit may control, on the basis of one kind of input image, enhancement processing on another kind of input image.

Accordingly, the visibility can be prevented from being impaired.

An image processing method according to an embodiment of the present technology includes reading an input image. Moreover, enhancement processing is performed on low-frequency components that are a range lower than a predetermined spatial frequency in an input image, enhancement processing is performed on high-frequency components that are a spatial frequency range higher than the low-frequency components in the input image, and an input image having the low-frequency components and the high-frequency components each subjected to enhancement processing is output.

An endoscope system according to an embodiment of the present technology includes an endoscope apparatus and an image processing apparatus.

The endoscope apparatus includes an endoscope provided with an objective lens at a distal end of an insertion portion to be inserted into a body cavity, and an imaging unit that captures an optical image formed by the objective lens and outputs the optical image as an image signal, the optical image being input from the endoscope.

The image processing apparatus includes an image reading unit that reads the image signal, and an enhancement processing unit that performs enhancement processing on low-frequency components that are a range lower than a predetermined spatial frequency in the image signal, performs enhancement processing on high-frequency components that are a spatial frequency range higher than the low-frequency components in the image signal, and outputs the image signal having the low-frequency components and the high-frequency components each subjected to enhancement processing.

### Brief Description of Drawings

[Fig. 1] A diagram showing an overview of laparoscopic surgery.
[Fig. 2] A block diagram showing a configuration example of an endoscope system to which the present technology is applied.
[Fig. 3] A block diagram showing another configuration example of an endoscope apparatus of Fig. 2.
[Fig. 4] An image diagram showing a spatial frequency spectrum of superficial blood vessels and deep blood vessels.
[Fig. 5] A graph result when frequency characteristics in regions of superficial blood vessels and deep blood vessels in an image captured at an angle of view of Fig. 4 is analyzed.
[Fig. 6] A schematic configuration diagram of a serial-type image processing apparatus according to a first embodiment of the present technology.
[Fig. 7] A flow of an image processing method according to the first embodiment of the present technology.
[Fig. 8] An example of an image processed by the image processing method of Fig. 6.
[Fig. 9] A schematic configuration diagram of a parallel-type image processing apparatus according to the first embodiment of the present technology.
[Fig. 10] A schematic configuration diagram of an image processing apparatus according to a second embodiment of the present technology.
[Fig. 11] A flow of an image processing method according to the second embodiment of the present technology.
[Fig. 12] A configuration diagram showing the schematic configuration of Fig. 10 more specifically.
[Fig. 13] An example of an image processed by the image processing method of Fig. 10.
[Fig. 14] A diagram for describing a configuration example of a general-purpose personal computer.

### Mode(s) for Carrying Out the Invention

Hereinafter, embodiments according to the present technology will be described with reference to the drawings. It should be noted that the same reference signs denote those that have the same functions.

### <Overview of Endoscope System>

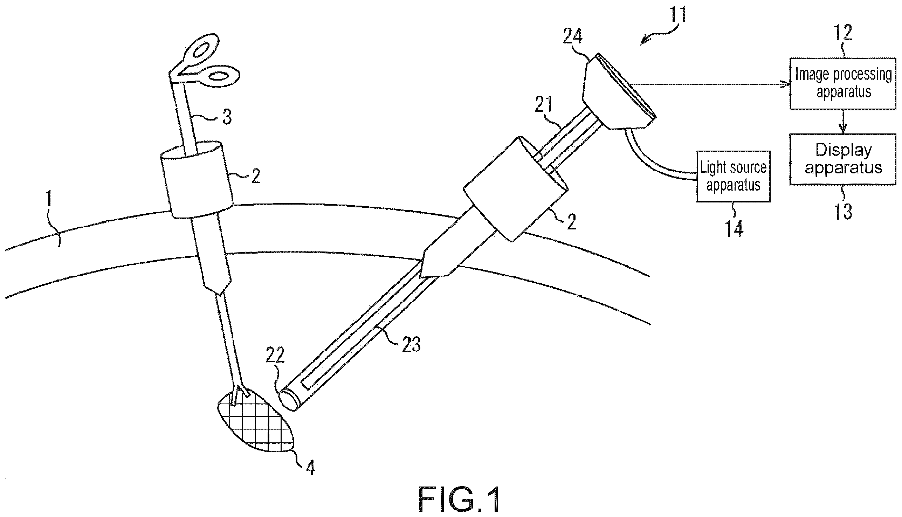

Fig. 1 is a diagram describing an overview of an endoscope system to which the present technology is applied.

In recent years, this type of endoscope system has been utilized in laparoscopic surgery performed instead of the conventional abdominal surgery in the clinical environment.

That is, as shown in Fig. 1, in laparoscopic surgery, in a case where abdominal surgery is performed, for example, instead of making an incision in an abdominal wall 1 for a laparotomy that has conventionally been performed, opening instruments called trocars 2 are attached at a plurality of positions of an abdominal wall 1 and a laparoscope (hereinafter, also referred to as endoscope apparatus or endoscope) 11 and a treatment instrument 3 are inserted into the body through holes provided in the trocars 2. Then, treatment such as ablation of an affected part 4 through the treatment instrument 3 is performed while viewing an image of an affected part (e.g., tumor) 4, which is imaged as video by an endoscope apparatus 11, in real time.

As to the endoscope apparatus 11 in a straight bar shape as shown in Fig. 1, a surgeon, assistant, scopist, or robot holds a head portion 24.

### <Configuration Example of Endoscope System>

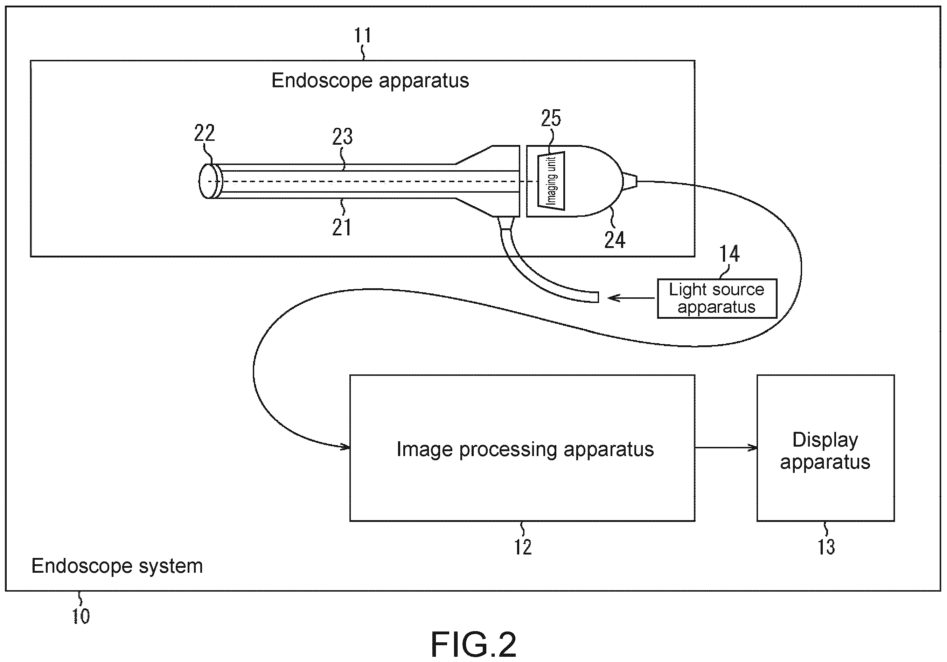

Here, a configuration example of the endoscope system that is the embodiment of the present technology will be described with reference to Fig. 2. This endoscope system 10 includes the endoscope apparatus 11, an image processing apparatus 12, and a display apparatus 13 (that displays an output image).

The endoscope apparatus 11 and the image processing apparatus 12 may be connected to each other via a cable or may be connected to each other wirelessly. Moreover, the image processing apparatus 12 may be disposed in a location remote from an operation room and connected via a network such as an in-house LAN and the Internet. The same applies to connection between the image processing apparatus 12 and the display apparatus 13.

The endoscope apparatus 11 includes a lens barrel portion 21 in a straight bar shape and the head portion 24. The lens barrel portion 21 is also referred to as a telescope or a rigid tube. The lens barrel portion 21 has a length of about several tens of centimeters. One end of the lens barrel portion 21, which is to be inserted into the body, is provided with an objective lens 22. Another end of the lens barrel portion 21 is connected to the head portion 24. An optical lens portion 23 of a relay optical system is provided inside the lens barrel portion 21. It should be noted that the shape of the lens barrel portion 21 is not limited to the straight bar shape.

The lens barrel portion 21 is roughly classified into a forward-viewing endoscope in which the lens barrel axis of Fig. 2 is equal to the optical axis and an oblique-viewing endoscope in which the lens barrel axis and the optical axis form a predetermined angle. The lens barrel portion 21 of Fig. 2 is an example of the forward-viewing endoscope of them.

The head portion 24 includes a built-in imaging unit 25. The imaging unit 25 includes an image pickup device such as a complementary metal oxide semiconductor (CMOS) image sensor. The imaging unit 25 converts an optical image of an affected part, which is input from the lens barrel portion 21, into image signals at a predetermined frame rate.

Moreover, a light source apparatus 14 is connected to the endoscope apparatus 11. Supplied with a light source necessary for imaging, the light source apparatus 14 illuminates the affected part 4. At this time, the light source apparatus 14 is capable of switching light having various wavelengths and emitting light and also capable of emitting, in addition to normal light, special light for identifying especially the affected part 4. Therefore, for an image captured by the imaging unit 25, image signals of the special light can also be captured in addition to image signals of the normal light. Although not shown in the figure, the endoscope apparatus 11 may be provided with a plurality of light source apparatuses 14 and light having various wavelengths may be emitted at the same time. In this embodiment, the endoscope apparatus 11 outputs to the image processing apparatus 12 at least one or a plurality of kinds of images of a white light image illuminated in white, a narrow band image illuminated with narrow-band light, a fluorescent image illuminated with excitation light, or the like as an input image.

In the endoscope apparatus 11, an optical image of the affected part 4, the light of which is collected by the objective lens 22, is made incident upon the imaging unit 25 of the head portion 24 via the optical lens portion 23, converted into image signals at a predetermined frame rate by the imaging unit 25, and output to the image processing apparatus 12 at the subsequent stage. Moreover, it is assumed that the head portion 24 is configured to provide the image processing apparatus 12 with information such as the type of light emitted by the light source apparatus 14, the diameter of the objective lens 22, and the diameter of the optical lens portion 23 as condition information.

For this condition information, a part (not shown) of the head portion 24 may be provided with a configuration by which the user inputs the condition information in advance and an image processing apparatus 122 may be provided with the condition information from this part. Moreover, the image processing apparatus 12 may be configured to recognize the condition information by itself by analyzing captured image signals at the image processing apparatus 12.

Here, the description will be continued on the assumption that the condition information is input into the image processing apparatus 12 by either one of the methods. It should be noted that the information about the type of light supplied to the image processing apparatus 12 from the light source apparatus 14 may be configured to be directly supplied to the image processing apparatus 12 from the light source apparatus 14.

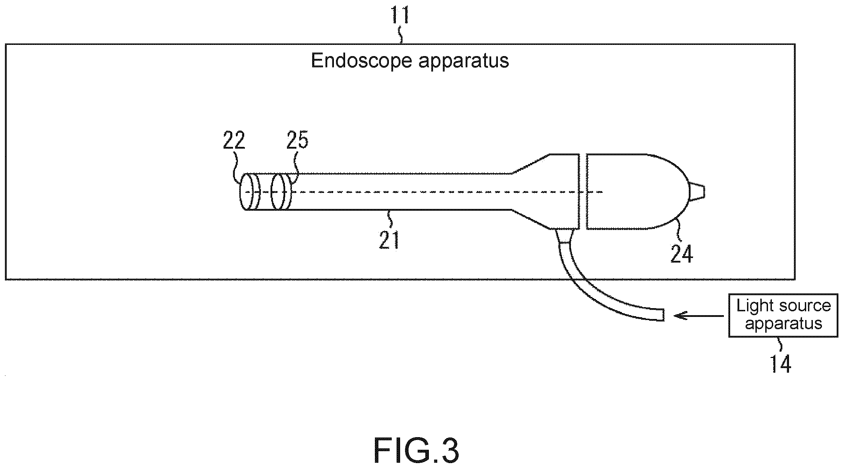

Fig. 3 shows another configuration example of the endoscope apparatus 11. As shown in the figure, the imaging unit 25 may be disposed immediately after the objective lens 22 and the optical lens portion 23 inside the lens barrel portion 21 may be omitted.

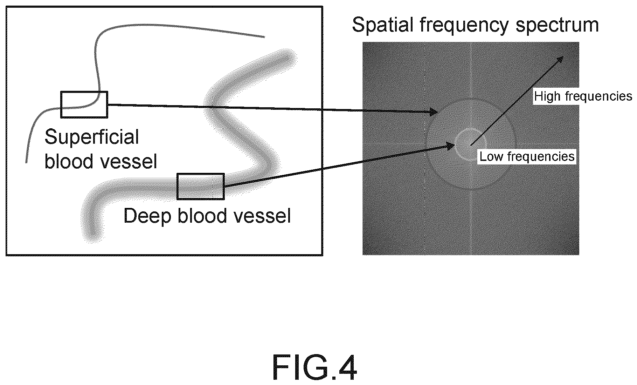

Fig. 4 is an image diagram of a medical image showing a spatial frequency spectrum of superficial blood vessels (superficial tissues) and deep blood vessels (deep tissues). As shown in the figure, the deep blood vessels correspond to low frequencies of the spatial frequency spectrum and the superficial blood vessels correspond to high frequencies of the spatial frequency spectrum, which are higher than the range of the deep blood vessels.

Since deep blood vessels are covered with biological membranes, fat, and the like, scattering blurring is greater and contains more low-frequency components than superficial blood vessels. Therefore, low-frequency enhancement is required for improving the visibility of deep blood vessels.

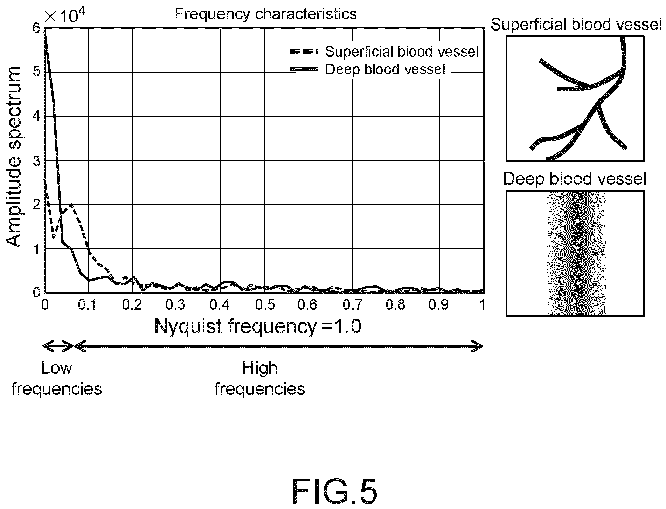

Fig. 5 is a graph result when frequency characteristics of regions of superficial blood vessels and deep blood vessels in an image captured at an angle of view of Fig. 4 is analyzed.

As shown in this figure, when the Nyquist frequency (frequency maximum value that can be represented when signals are sampled) is set to 1, the amplitude spectrum of the deep blood vessels is higher than the amplitude spectrum of the superficial blood vessels in the range of the Nyquist frequency that is equal to or higher than 0 and lower than 0.05.

Then, the amplitude spectrum of the superficial blood vessels is higher than the amplitude spectrum of the deep blood vessels in the range of the Nyquist frequency that is equal to or higher than 0.05 and lower than 0.17. Here, the frequency characteristics equal to or higher than the Nyquist frequency of 0.17 are negligible for distinguishing the regions of superficial blood vessels and deep blood vessels.

Therefore, in this example, (input image) signals in the range of the Nyquist frequency, which is equal to or higher than 0 and lower than 0.05, can be considered as low-frequency components and signals beyond the range can be considered as high-frequency components. In other words, in the input image, a range lower than a predetermined spatial frequency (Nyquist frequency of 0.05) is considered as low-frequency components and a spatial frequency range higher than the low-frequency components is considered as the high-frequency components.

Alternatively, signals equal to or higher than a predetermined amplitude spectrum (e.g., 2.0 × 10⁴) may be considered as low-frequency components and signals below the predetermined amplitude spectrum may be considered as high-frequency components.

The frequency characteristics vary depending on zoom-in/out of the endoscope apparatus 11. For example, the thickness of blood vessels with respect to the angle of view increases as the zoom-in level increases, and therefore the spatial frequency decreases. In contrast, the thickness of blood vessels with respect to the angle of view decreases as the zoom-out level increases, and therefore the spatial frequency increases. Therefore, in a case where zoom-in is performed, the filter size for low frequencies or high frequencies may be automatically increased accordingly, and in a case where zoom-out is performed, the filter size for low frequencies or high frequencies may be automatically decreased accordingly.

### <First Embodiment of Image Processing Apparatus>

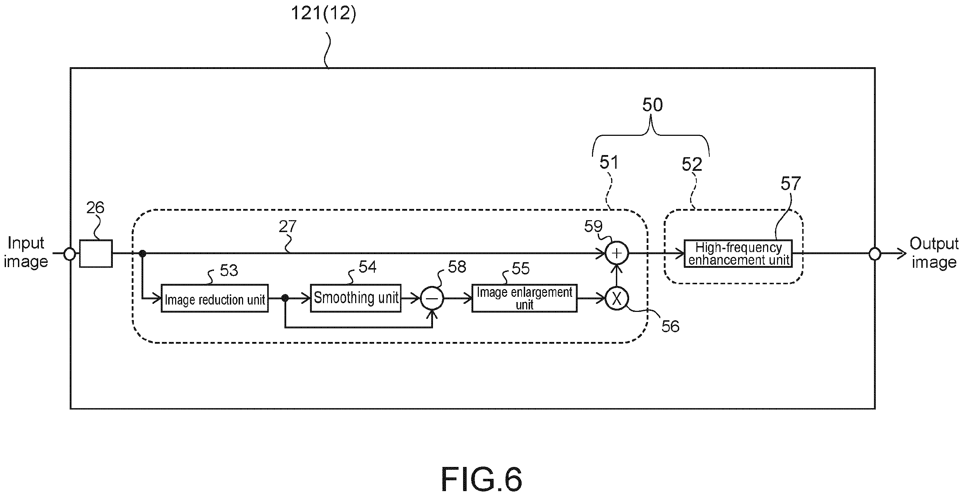

Fig. 6 is a schematic configuration diagram showing a serial-type image processing apparatus 121 as the image processing apparatus 12 according to this embodiment. As will be described later, the image processing apparatus 121 typically includes a computer having a CPU, a memory, and the like.

The image processing apparatus 121 includes an image reading unit 26 and an enhancement processing unit 50 as functional blocks of the CPU. The enhancement processing unit 50 performs enhancement processing on low-frequency components that are a range lower than the predetermined spatial frequency in the input image (in this embodiment, the medical image), performs enhancement processing on the high-frequency components that are a spatial frequency range higher than the low-frequency components in the input image, and outputs the input image having the low-frequency components and the high-frequency components each subjected to enhancement processing.

The enhancement processing unit 50 includes a low-frequency enhancement processing unit 51 that performs enhancement processing with respect to the low-frequency components of the input image and a high-frequency enhancement processing unit 52 that performs enhancement processing with respect to the high-frequency components of the input image. In this embodiment, by the low-frequency enhancement processing unit 51 and the high-frequency enhancement processing unit 52 arranged in series, the enhancement processing unit 50 is configured to combine a low-frequency-enhanced image that is an image having the low-frequency components subjected to enhancement processing with the input image and perform enhancement processing on the high-frequency components of the input image combined with the low-frequency-enhanced image.

The low-frequency enhancement processing unit 51 includes an image reduction unit 53, a smoothing unit 54, a difference processing unit 58, an image enlargement unit 55, a gain multiplication unit 56, and a composition processing unit 59. The high-frequency enhancement processing unit 52 includes a high-frequency enhancement unit 57.

The low-frequency enhancement processing unit 51 includes the image reduction unit 53, the smoothing unit 54, the image enlargement unit 55, and the gain multiplication unit 56 in series in the stated order.

The image reduction unit 53 reduces the input image acquired by the image reading unit 26 into an image having a predetermined low resolution.

The smoothing unit 54 smooths the image reduced at the image reduction unit 53, at a predetermined filter size (smoothing strength).

The difference processing unit 58 takes a difference between the output of the image reduction unit 53 and the output of the smoothing unit 54, to thereby acquire a difference image therebetween.

The image enlargement unit 55 increases the resolution of the difference image output from the difference processing unit 58. The increase rate is typically an increase rate corresponding to the reduction rate when the image reduction unit 53 performs reduction processing.

The gain multiplication unit 56 multiplies the image signals enlarged at the image enlargement unit 55 by a predetermined digital gain (coefficient). The digital gain adjusts (low-frequency) enhancement level and the value can be arbitrarily adjusted.

The composition processing unit 59 is provided in a bypass route 27 that directly connects the image reading unit 26 and the high-frequency enhancement processing unit 52 to each other. The composition processing unit 59 combines the input image acquired by the image reading unit 26 with the image output from the gain multiplication unit 56 and outputs the composite image to the high-frequency enhancement processing unit 52.

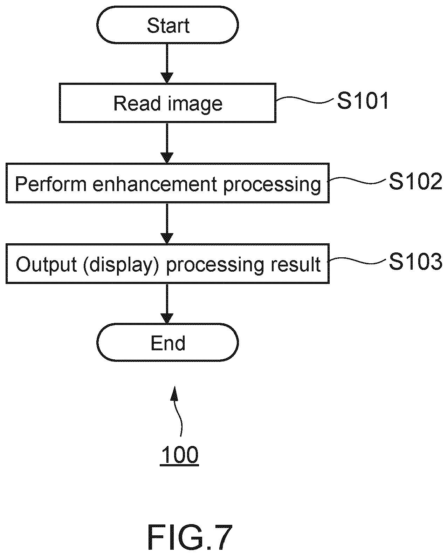

Fig. 7 is a flow of the image processing method performed in the image processing apparatus 121 according to the first embodiment of the present technology.

In Step S101 of this image processing method 100, the image reading unit 26 reads the input image (image signals output from the endoscope apparatus 11).

In Step S102, the low-frequency enhancement and the high-frequency enhancement for the input image are performed. As shown in Fig. 6, at the low-frequency enhancement processing unit 52, the input image is separated into two kinds. As to one input image, after the image reduction unit 53 performs resolution reduction processing, the smoothing unit 54 performs smoothing processing, and the difference processing unit 58 acquires a difference image between images before and after smoothing.

Since the reduction processing of the input image is performed before the input image is smoothed, the processing time required for smoothing can be reduced.

The acquired difference image is increased in resolution by the image enlargement unit 55 at the increase rate corresponding to the reduction rate in the image reduction unit 53. Accordingly, the original resolution before reduction can be restored. This image is multiplied by a predetermined digital gain at the gain multiplication unit 56 as a low-frequency enhancement component image, and then combined with the other input image separated into the two kinds (image input into the composition processing unit 59 from the image reading unit 26 via the bypass route 27) at the composition processing unit 59.

By this processing, components higher than an arbitrary spatial frequency can be enhanced in a manner that depends on the smoothing strength. The high-frequency enhancement unit 57 enhances superficial blood vessels by, for example, edge enhancement processing using the image after the low-frequency enhancement.

Subsequently, in Step S103, the image subjected to enhancement processing at the enhancement processing unit 50 is output to the display apparatus 13 and the display apparatus 13 displays the processing result.

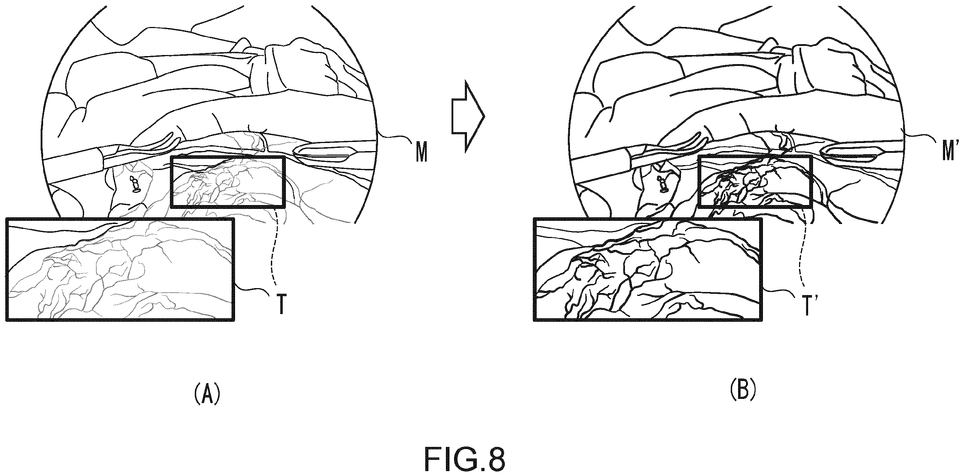

An example of the image processed by the image processing method 100 is shown in Fig. 8. In this example, a 4K image (3840 × 2160 pixels) is input into the low-frequency enhancement processing unit 51 as the input image (Fig. 8 (A)) and is reduced into an image having a 1/64 resolution at the image reduction unit 53. Thereafter, it is smoothed with a Gaussian filter having a filter size of 27 pixels at the smoothing unit 54, and then a low-frequency enhancement processing image is acquired by multiplying the difference image enlarged 64 times at the image enlargement unit 55 by the double gain. Next, the high-frequency enhancement processing unit 52 acquires the image to which an edge enhancement filter is applied for enhancing the high-frequency components.

In the output image (Fig. 8 (B)) obtained by the above-mentioned band enhancement processing at the two stages, the contrast enhancement (M') of blood vessels covered with membranes and the sharpness (T') of thin superficial blood vessels have been increased as compared to blood vessels M covered with membranes in Fig. 8 (A) and thin superficial blood vessels T.

It should be noted that in general, as the smoothing filter size is increased, it leads to a problem in that the image processing cost increases, and therefore it is inappropriate for processing a medical image for surgery that requires the real time property. In the above-mentioned example, if the reduction processing is not performed, the smoothing filter size that is eight times as large as the original size and exceeds 200 pixels is required for performing low-frequency enhancement at the same level. It is very difficult to apply the filter having this size to a 4K image and perform real time processing.

In contrast, in accordance with this embodiment, as in the example of Fig. 8, the 4K input image is reduced into 1/64, which can reduce the size of the smoothing filter at the subsequent stage to a small size, 27 pixels, and high speed processing is achieved. In addition, processing that does not enhance high-frequency noise components contained in the input image is possible by performing the reduction processing.

It should be noted that the above-mentioned reduction rate and increase rate are not limited thereto, and for example, the image may be converted into full HD (1920 × 1080 pixels) from 4K.

As described above, in accordance with this embodiment, the contrast of deep tissues is increased by the low-frequency enhancement processing. In addition, by also using the high-frequency enhancement processing, not only the visibility of deep tissues but also the visibility of superficial tissue structures can also be improved. Moreover, since the image processing result can be displayed to the surgeon in real time, the visibility of deep biological tissues covered with biological membranes, fat, and the like can be improved and more safe surgery support can be achieved.

The enhancement processing unit 50 combines the low-frequency-enhanced image that is an image having the low-frequency components subjected to enhancement processing with the input image and performs enhancement processing on the high-frequency components of the input image combined with the low-frequency-enhanced image.

By separating the input image into the two kinds as in Fig. 6, the high-frequency components contained in the original image can be left in the composite image after the low-frequency enhancement, and therefore the high-frequency enhancement at the subsequent stage can be performed. In this embodiment, the high-frequency enhancement is performed after the low-frequency enhancement is performed, though they can be performed also in reverse order.

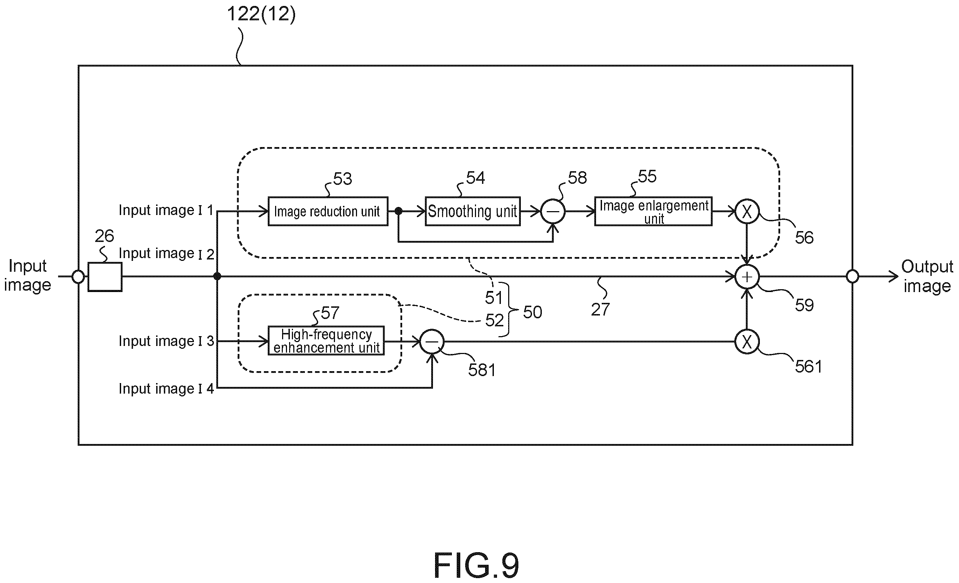

In addition, not only the serial-type flow in which the low-frequency enhancement and the high-frequency enhancement shown in Fig. 6 are continuously performed, but also a processing flow in which the low-frequency enhancement and the high-frequency enhancement are processed in parallel and finally combined as in Fig. 9 can be performed.

### <Second Embodiment of Image Processing Apparatus>

Fig. 9 is a schematic configuration diagram showing a parallel-type image processing apparatus 122 as an image processing apparatus 12 according to this embodiment. Hereinafter, configurations different from those of the first embodiment will be mainly described and configurations similar to those of the first embodiment will be denoted by similar reference signs and descriptions thereof will be omitted or simplified.

Here, unlike the serial-type image processing apparatus 121 according to the first embodiment, the low-frequency enhancement processing unit 51 and the high-frequency enhancement processing unit 52 are arranged in parallel with respect to the image reading unit 26 in the enhancement processing unit 50. The enhancement processing unit 50 is configured to combine the low-frequency-enhanced image and the high-frequency enhanced image with the input image.

In the low-frequency enhancement processing unit 51, the image reduction unit 53, the smoothing unit 54, the difference processing unit 58, the image enlargement unit 55, and the gain multiplication unit 56 are connected in series. The high-frequency enhancement processing unit 52 includes the high-frequency enhancement unit 57. The image processing unit 122 further includes a difference processing unit 581 that takes a difference between an image before the high-frequency enhancement and an image after the high-frequency enhancement and a gain multiplication unit 561 that multiplies an output image (difference image) of the difference processing unit 581 by a predetermined digital gain. The image subjected to high-frequency enhancement processing is combined with the input image and the image subjected to low-frequency enhancement processing at the composition processing unit 59.

In this manner, the image processing apparatus 122 according to this embodiment combines the low-frequency-enhanced image that is an image having the low-frequency components subjected to enhancement processing and the high-frequency enhanced image that is an image with the high-frequency components subjected to enhancement processing with the input image.

In this image processing apparatus 122, the input image is separated into four kinds (input image I1, input image 12, input image 13, and input image 14) at the image reading unit 26.

The input image I1 is used for generating an enhancement component image of the low-frequency enhancement processing unit 51. The low-frequency enhancement processing unit 51 generates a low-frequency enhancement component image with respect to the input image on the basis of the input image I1.

The input image I2 is input into the composition processing unit 59 via the bypass route 27.

The input image I3 is used in the high-frequency enhancement processing unit 52 and the input image I4 is input into the difference processing unit 581. The high-frequency enhancement processing unit 52 generates a high-frequency enhancement component image with respect to the input image on the basis of the input image I3 and the input image 14.

After the low-frequency enhancement component image and the high-frequency enhancement component image are multiplied by a suitable gain by the gain multiplication units 56 and 561, respectively, the low-frequency enhancement component image and the high-frequency enhancement component image are combined with the input image I2 at the composition processing unit 59 and output to the display apparatus 13.

Also in this embodiment, actions and effects similar to those of the above-mentioned first embodiment can be obtained. In accordance with this embodiment, the low-frequency enhancement processing and the high-frequency enhancement processing of the input image are respectively performed in parallel, and therefore the time required for the image processing can be reduced. Accordingly, the real time property of the image display in the display apparatus 13 is improved.

### <Third Embodiment of Image Processing Apparatus>

In the above-mentioned first and second embodiments, the low-frequency enhancement component image is enhanced with the equal gain at the gain multiplication unit 56 in the low-frequency enhancement processing unit 51. However, an effect of further improving the visibility can be obtained by controlling the enhancement gain in accordance with characteristics of the input image.

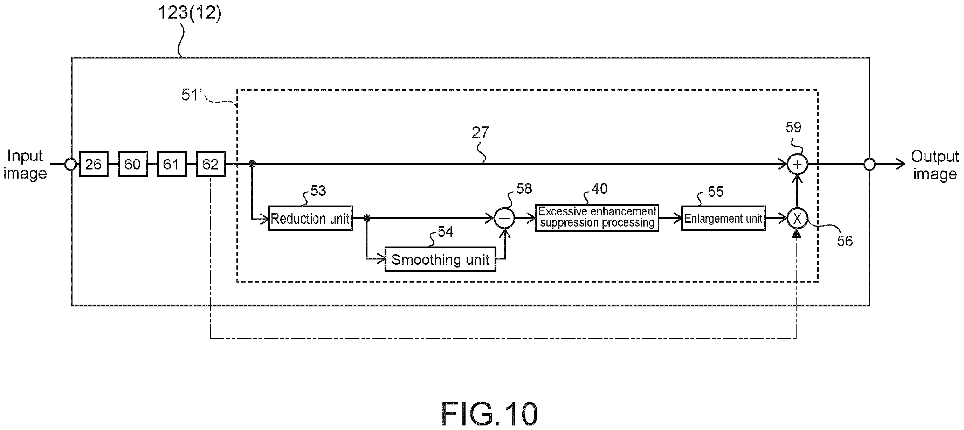

Fig. 10 is a schematic configuration diagram of an image processing apparatus 123 according to a third embodiment of the present technology. Hereinafter, configurations different from those of the first embodiment will be mainly described and configurations similar to those of the first embodiment will be denoted by similar reference signs and descriptions thereof will be omitted or simplified.

This image processing apparatus 123 includes the image reading unit 26, a luminance/chrominance separation unit (separation processing unit) 60, a luminance/chrominance discrimination unit 61, a gain adjustment unit 62, and a low-frequency enhancement processing unit 51'. The low-frequency enhancement processing unit 51' is different from that of the first embodiment in that the low-frequency enhancement processing unit 51' includes an excessive enhancement suppression processing unit 40 between the difference processing unit 58 and the image enlargement unit 53.

It should be noted that the luminance/chrominance separation 60 and the luminance/chrominance discrimination unit 61 may be constituted by a single functional block. Moreover, although not shown in the figure, the image processing apparatus 123 includes a high-frequency enhancement processing unit having the high-frequency enhancement unit 57. The high-frequency enhancement processing unit may be configured in series with the low-frequency enhancement processing unit as in the first embodiment or may be configured in parallel with the low-frequency enhancement processing unit as in the second embodiment.

The luminance/chrominance separation unit 60 separates an input image into a luminance component image and a chrominance component image. The gain adjustment unit 62 selects pixels to be enhanced and pixels not to be enhanced from the luminance component image and the chrominance component image and adjusts gains to be multiplied with respect to the pixels to be enhanced and the pixels not to be enhanced. The excessive enhancement suppression processing unit 40 has a function of reducing the enhancement level of an excessively enhanced portion (pixel region) of the low-frequency-enhanced image (difference image) obtained in the difference processing unit 40.

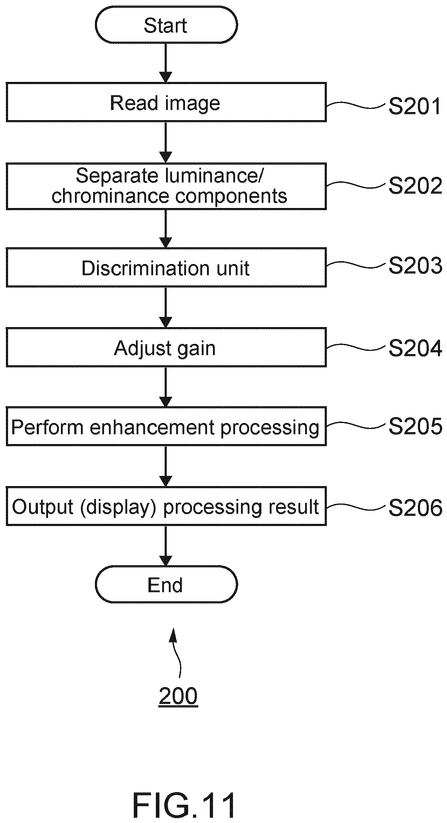

Fig. 11 is a flow of the image processing method performed in the image processing apparatus 123.

In Step S201 of this image processing method 200, as in Step S101, the image reading unit 26 reads an input image (e.g., a 4K image) in the image processing apparatus 12. Subsequently, in Step S202, the luminance/chrominance separation unit 60 separates a luminance component and chrominance components of the input image. Subsequently, in Step S203, the luminance/chrominance discrimination unit 61 discriminates the respective components.

Subsequently, in Step S204, the gain adjustment unit 62 adjusts the gain of the gain multiplication unit 56 on the basis of the discriminated component information. Subsequently, in Step S205, the enhancement processing unit 51' performs enhancement processing of the input image. In Step 206, the image subjected to enhancement processing is output to the display apparatus 13 and the display apparatus 13 displays the processing result.

In the low-frequency enhancement processing unit 51', the input image is separated into two kinds (see Fig. 10). As to one input image, after the image reduction unit 53 performs resolution reduction processing (e.g., 1/64 times), the smoothing unit 54 performs smoothing processing and the difference processing unit 58 takes a difference between images before and after smoothing to acquire a difference image.

Here, in the smoothing unit 54, as the degree of shading of the image is greater (as the smoothing is stronger), the enhancement components for the low-frequency components become larger. In the acquired difference image, the value of the enhancement components is greater in a portion in which the gradient of smoothing is larger, and therefore the excessive enhancement suppression processing unit 58 performs processing of suppressing excessive enhancement (e.g., 1/64 times).

The resolution of the image for which the excessive enhancement is suppressed is increased at an increase rate corresponding to the reduction rate in the image reduction unit 53. This enhancement component image is multiplied by the digital gain subjected to gain adjustment by the gain adjustment unit 62 described in the gain multiplication unit 56 and is combined with the input image input into the composition processing unit 59 via the bypass route 27. This processing can enhance components higher than an arbitrary spatial frequency in a manner that depends on the smoothing strength.

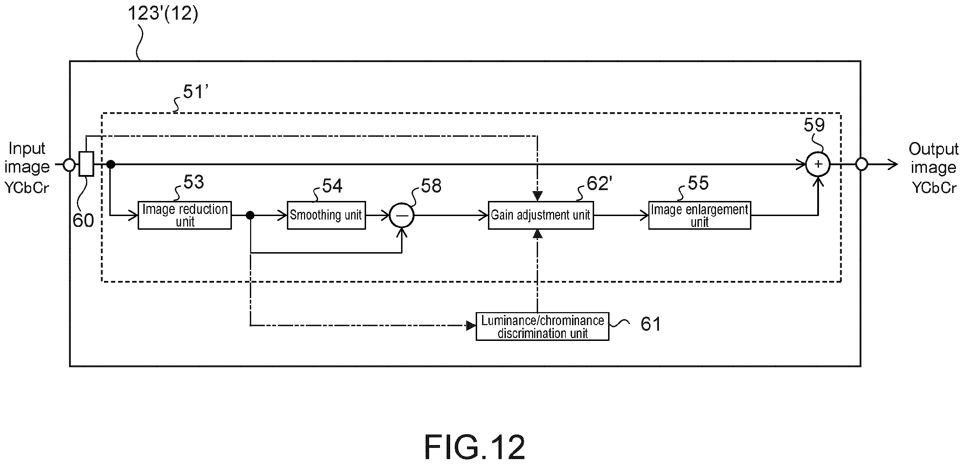

Fig. 12 is a configuration diagram of an image processing apparatus 123' that is a specific example of the image processing apparatus 123 of Fig. 10. Hereinafter, configurations different from those of Fig. 10 will be described.

Here, the low-frequency enhancement processing unit 51' includes the image reduction unit 53, the smoothing unit 54, the difference processing unit 58, a gain adjustment unit 62', the image enlargement unit 55, and the composition processing unit 59. The gain adjustment unit 62' multiplies, for each of the luminance component and the chrominance components, a difference image output from the difference processing unit 58 by a predetermined gain and outputs it to the image enlargement unit 55.

The input image (YCbCr) is a color space represented by a luminance signal Y and two color difference signals Cb and Cr. In this embodiment, the low-frequency enhancement is applied to each component of the luminance component (Y) and the chrominance components (Cb, Cr) of the input image.

For example, in a case where only the luminance component is enhanced without enhancing the chrominance components, the blood vessel portions looks darker and less red. Since the colors of the blood vessels are important for observing a blood circulation state, it is favorable not to reduce the red color.

As one of them, in a case where only the chrominance components are enhanced without enhancing the luminance component, the colors are enhanced but the structure is not enhanced, and therefore the visibility is impaired. In view of this, by enhancing both of the luminance component and the chrominance components, the contrast of colors of the blood vessels and the like is increased while enhancing the structure, and the visibility of deep blood vessels, lymph nodes, and the like can be improved.

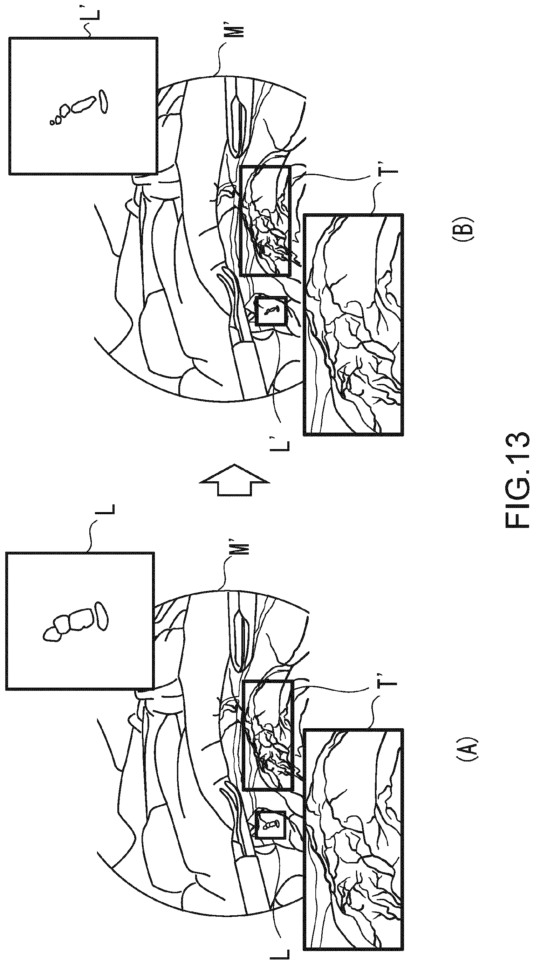

Moreover, separating the luminance component and the chrominance components enables the independent enhancement gain depending on each of the characteristics to be controlled. An example of the image subjected to enhancement processing by the image processing method 200 is shown in Fig. 13 (B). It can be seen that while increasing the contrast (M') of the blood vessels and the sharpness (T') of thin superficial blood vessels, an image (L') in which a spread (L) of bright points is controlled is obtained as compared to Fig. 13 (A) (identical to Fig. 8 (B)) processed by the image processing method 100.

### [Enhancement Control of Luminance Component]

The luminance/chrominance discrimination unit 61 detects pixels to be enhanced and pixels not to be enhanced on the basis of luminance (Y) information of the reduced image. For example, enhancement of pixels having high luminance where the halation has occurred is set to be smaller. Or, excessive enhancement is suppressed by setting enhancement of blood portions having low luminance to be smaller.

When the enhancement processing is applied, excessive enhancement can occur in a high-luminance or low-luminance portion. For example, the bright points can be more spread or the blood can look darker. In view of this, excessive enhancement can be suppressed by performing enhancement control not to enhance dark and bright portions with respect to the luminance components.

### [Enhancement Control of Chrominance Components]

The luminance/chrominance discrimination unit 61 detects pixels to be enhanced and pixels not to be enhanced on the basis of chrominance (Cb, Cr) information of the reduced image. For example, processing of reducing enhancement for portions where Cb and Cr signals have high values by chromatic aberration is performed. If the chromatic aberration portion in the image is enhanced, the color shifting becomes more noticeable and the visibility is impaired. In view of this, the excessive enhancement of the chromatic aberration can be suppressed by adding the enhancement control not to perform excessive enhancement of the chrominance components.

In addition, the visibility of biological tissues can be further improved by performing color enhancement control. For example, since arteries (e.g., reddish) and veins (e.g., bluish) have different colors, it is sufficient to enhance especially the color of each of them for enhancing the arteries and/or veins.

Moreover, since deep blood vessels are less vivid than superficial blood vessels, analyzing the color information can selectively enhance chrominance components of the deep blood vessels to thereby improve the visibility. In addition, medical images show artificial objects (e.g., forceps) such as treatment instruments other than a living body. Since the colors (e.g., green or silver color) of artificial objects are greatly different from those of biological tissues, they can be separated by color.

Therefore, control not to enhance those that do not have the living body's colors can be performed.

As described above, in accordance with this embodiment, the contrast of the deep tissues is increased and in addition, by also using the other enhancement processing (high-frequency enhancement processing), not only the visibility of deep tissues but also the visibility of superficial tissue structures can be improved at the same time. In addition, since the gain adjustment is also used, it is possible to enhance the structures and increase the color contrast of blood vessels and the like by enhancing the luminance component and the chrominance components and to improve the visibility of deep blood vessels, lymph nodes, and the like.

By displaying the image processing result according to this embodiment to the surgeon in real time, the visibility of deep biological tissues covered with biological membranes, fat, and the like can be improved and more safe surgery support can be achieved.

### <Fourth Embodiment of Image Processing Apparatus>

In addition to the single medical image (white light image) captured (illuminated) in white light, other kinds of medical images such as a narrow band image illuminated with narrow-band light and a fluorescent image illuminated with excitation light may be utilized as the input image. Although not limited thereto, a case where two kinds of images, white light image and fluorescent image, are used as the input images will be described.

Since scattering blurring is greater in the fluorescent image with excitation light than a normal white light image, the contrast enhancement effect by the low-frequency enhancement is larger. Moreover, enhancement processing using a plurality of kinds of images can remove superficial blurring while increasing the contrast of deep tissues by employing enhancement to correct superficial fluorescence blurring, lens aberration, and the like.

Therefore, the visibility of the medical image can be improved by performing enhancement processing by the use of both the white light image and the fluorescent image. Hereinafter, application examples will be shown.

It should be noted that image discrimination may be, for example, performed at the luminance/chrominance discrimination unit 61 in the image processing apparatus 123 (123') or another discrimination unit for image discrimination may be further provided. Alternatively, the respective images may be processed by a common low-frequency enhancement processing unit or may be processed by different low-frequency enhancement processing units for each image.

### (Application Example 1)

The low-frequency enhancement processing is performed on both of the white light image and the fluorescent image and they are displayed on a plurality of monitors or a single monitor by picture in picture (PinP). Accordingly, both of the white light/fluorescent images can be observed at the same time in a high visibility state.

### (Application Example 2)

Both of the white light image and the fluorescent image are subjected to low-frequency enhancement processing and displayed with the fluorescent image superimposed on the white light image. Accordingly, the high-visibility fluorescent image can be observed while improving the visibility of surrounding tissues that do not glow.

### (Application Example 3)

A region where fluorescence occurs is extracted from the fluorescent image and this region can be enhanced with the white light image. Accordingly, the surgeon can perform surgical procedure while checking the states of actual bile duct, blood vessels, greater omentum (fat tissues), and the like, which are invisible when something is superimposed thereon.

### (Application Example 4)

It is difficult to visually recognize blood vessels under fat even by checking the fluorescent image. In view of this, fat may be detected from the white light image. Then, in a case where fluorescence occurs in that region, only the fluorescent image under fat may be specifically enhanced by increasing the enhancement level of the fluorescent image.

Organs in which fluorescent dye (ICG) is apt to accumulate, such as a liver, strongly emit light after a while, and the visibility of a region wished to be actually viewed can be impaired. In view of this, an organ is identified on the basis of the white light image and control to weaken the enhancement is performed on an organ that accumulates fluorescent dye and becomes very bright, such as a liver. In this way, the visibility can be prevented from being impaired. That is, in a case where two (or more) kinds of images such as a white light image, a narrow band image, and a fluorescent image, are input, the enhancement processing unit 50 controls, on the basis of one (one kind of) image, enhancement processing of another (another kind of) image.

### (Application Example 5)

If blood mixed with fluorescent dye flows out, the periphery becomes very bright. In view of this, when the blood is identified on the basis of the white light image and control to weaken the enhancement is performed on the fluorescence of the blood portion. In this way, the observation can be performed while preventing the field of view from becoming too bright.

As described above, by the low-frequency enhancement in the technology of this embodiment, the contrast of the deep tissues is enhanced and in addition, by also using the enhancement processing (high-frequency enhancement processing), not only the visibility of deep tissues but also the visibility of superficial tissue structures can be improved at the same time. In addition, with enhancement processing using a plurality of kinds of images, superficial blurring can be removed while increasing the contrast of deep tissues by employing enhancement to correct superficial fluorescence blurring, lens aberration, and the like.

By displaying the image processing result according to this embodiment to the surgeon in real time, the visibility of deep biological tissues covered with biological membranes, fat, and the like can be improved and more safe surgery support can be achieved.

### <Example of Execution by Software>

By the way, the series of processing by the image processing apparatus 12 described above can be executed by hardware. However, the series of processing can also be executed by software. In a case where the series of processing is executed by the software, a program configuring the software is installed from a recording medium to a computer incorporated in dedicated hardware, or, for example, a general-purpose personal computer capable of executing various functions by installing various programs.

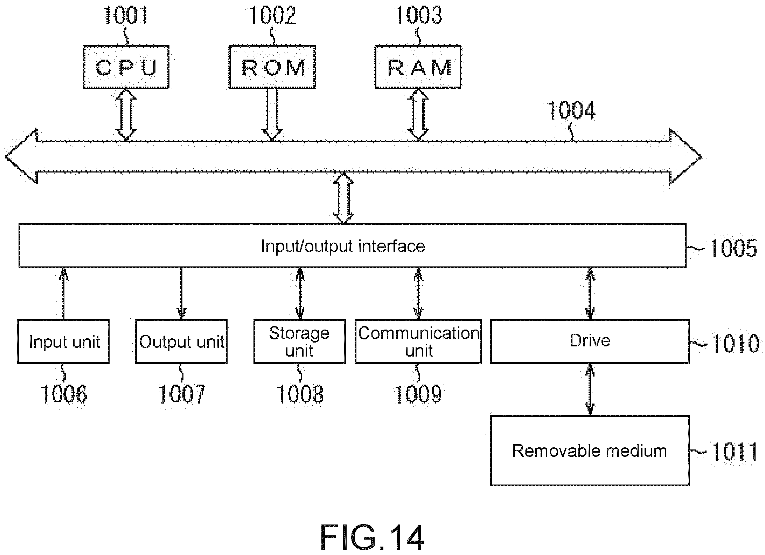

Fig. 14 shows a configuration example of the general-purpose personal computer. The personal computer incorporates a central processing unit (CPU) 1001. The CPU 1001 is connected to an input/output interface 1005 via a bus 1004. The bus 1004 is connected to a read only memory (ROM) 1002 and a random access memory (RAM) 1003.

The input/output interface 1005 is connected to an input unit 1006 including input devices such as a keyboard, a mouse for a user to input an operation command, an output unit 1007 for outputting to a display device a processing operation screen and an image of a processing result, a storage unit 1008 including a hard disk drive and the like for storing programs and various data, and a communication unit 1009 including a local area network (LAN) adapter and the like for executing communication processing via a network typified by the Internet. In addition, a drive 1010 is connected for reading data from and writing data to a removable medium 1011 such as a magnetic disk (including flexible disk), an optical disk (including compact disc-read only memory (CD-ROM), a digital versatile disc (DVD)), a magneto optical disk (including MiniDisc (MD)), and a semiconductor memory.

The CPU 1001 executes various types of processing in accordance with a program stored in the ROM 1002, or a program read from the removable medium 1011, such as the magnetic disk, the optical disk, the magneto optical disk, and the semiconductor memory, to be installed to the storage unit 1008, and loaded to the RAM 1003 from the storage unit 1008. Data necessary for the CPU 1001 to execute the various types of processing is also stored in the RAM 1003 as appropriate.

In the computer configured as described above, for example, the CPU 1001 loads the program stored in the storage unit 1008 to the RAM 1003 via the input/output interface 1005 and the bus 1004 to execute the series of processing described above.

The program executed by the computer (CPU 1001) can be provided, for example, by being recorded in the removable medium 1011 as a package medium or the like. In addition, the program can be provided via a wired or wireless transmission medium such as a local area network, the Internet, and digital satellite broadcasting.

In the computer, the program can be installed to the storage unit 1008 via the input/output interface 1005 by mounting the removable medium 1011 to the drive 1010. In addition, the program can be installed to the storage unit 1008 by receiving with the communication unit 1009 via the wired or wireless transmission medium. Moreover, the program can be installed in advance to the ROM 1002 and the storage unit 1008.

It should be noted that the program executed by the computer can be a program by which the processing is performed in time series along the order described herein, and can be a program by which the processing is performed in parallel or at necessary timing such as when a call is performed.

In addition, herein, a system means an aggregation of a plurality of constituents (apparatus, module (component), and the like), and it does not matter whether or not all of the constituents are in the same cabinet. Therefore, a plurality of apparatuses that is accommodated in a separate cabinet and connected to each other via a network and one apparatus that accommodates a plurality of modules in one cabinet are both systems.

It should be noted that the embodiments of the present technology are not limited to the embodiments described above, and various modifications can be made without departing from the gist of the present technology.

For example, the present technology can employ a configuration of cloud computing that shares one function in a plurality of apparatuses via a network to process in cooperation.

In addition, each step described in the above flowcharts can be executed by sharing in a plurality of apparatuses, other than being executed by one apparatus.

Moreover, in a case where a plurality of processes is included in one step, the plurality of processes included in the one step can be executed by sharing in a plurality of apparatuses, other than being executed by one apparatus.

### <Modified Examples>

### (Modified Example 1)

By using data learned from medical images the luminance/chrominance discrimination unit 61 according to the third embodiment of the present technology described above, artifacts, biological tissues, deep blood vessels, superficial blood vessels, and the like can be more easily identified. A region to be focused on can be further enhanced by increasing the enhancement gain for a region wished to be enhanced in the gain adjustment unit 62 or 62' on the basis of a discrimination result.

In this case, the luminance/chrominance discrimination unit 61 includes a database for storing mass training data including image portions to be enhanced (e.g., biological tissues and blood vessels) or image portions not to be enhanced (e.g., artificial objects, etc.), a control unit that determinates or extracts an image region to be enhanced or an image region not to be enhanced from the input image on the basis of the database, and the like.

Alternatively, instead of the luminance discrimination unit 61, an AI sensor that selectively enhances and outputs an image to be enhanced may be mounted on the endoscope apparatus 11 (e.g., the imaging unit 25).

### (Modified Example 2)

Various smoothing filters including a moving average filter, a median filter, a bilateral filter, and the like other than the Gaussian filter can be applied as the smoothing unit 54 provided in the low-frequency enhancement processing unit 51 or 51' in each embodiment of the present technology.

### (Modified Example 3)

In addition, in each of the above-mentioned embodiments, the medical image has been described as an example of the input image, though not limited thereto. For example, the present technology can also be applied to enhancement processing for images and the like of tissues of living things and plants.

It should be noted that the present technology may also take the following configurations.
(1) An image processing apparatus, including
   an enhancement processing unit that performs enhancement processing on low-frequency components that are a range lower than a predetermined spatial frequency in an input image, performs enhancement processing on high-frequency components that are a range higher than the low-frequency components in the input image, and outputs the input image having the low-frequency components and the high-frequency components each subjected to enhancement processing.
(2) The image processing apparatus according to (1), in which
   the enhancement processing unit includes a low-frequency enhancement processing unit that performs enhancement processing with respect to the low-frequency components of the input image, and
   the low-frequency enhancement processing unit smoothes the input image and obtains a difference image on the basis of a difference between the input image after smoothing and the input image before smoothing.
(3) The image processing apparatus according to (1) or (2), in which
   the low-frequency enhancement processing unit performs reduction processing on resolution of the input image at a predetermined reduction rate before the input image is smoothed.
(4) The image processing apparatus according to any one of (1) to (3), in which
   the low-frequency enhancement processing unit increases resolution of the difference image at an increase rate corresponding to the predetermined reduction rate after the input image is smoothed.
(5) The image processing apparatus according to (4), in which
   the low-frequency enhancement processing unit outputs a low-frequency-enhanced image obtained by multiplying an image having the resolution increased by a predetermined coefficient and combining the multiplied image with the input image.
(6) The image processing apparatus according to any one of (1) to (5), in which
   the enhancement processing unit combines a low-frequency-enhanced image, which is an image having the low-frequency components subjected to enhancement processing, with the input image and performs enhancement processing on the high-frequency components of the input image combined with the low-frequency-enhanced image.
(7) The image processing apparatus according to any one of (1) to (5), in which
   the enhancement processing unit combines a low-frequency-enhanced image, which is an image having the low-frequency components subjected to enhancement processing, and a high-frequency enhanced image, which is an image with the high-frequency components subjected to enhancement processing, with the input image.
(8) The image processing apparatus according to any one of (1) to (7), in which
   the low-frequency enhancement processing unit includes
   a separation processing unit that separates the input image into a luminance component image and a chrominance component image, and
   a gain adjustment unit that selects, from the luminance component image and the chrominance component image, pixels to be enhanced and pixels not to be enhanced, and adjusts gains to be multiplied with respect to the pixels to be enhanced and the pixels not to be enhanced.
(9) The image processing apparatus according to any one of (1) to (8), in which
   the input image is a medical image.
(10) The image processing apparatus according to any one of (1) to (9), in which
   the input image includes at least one of a white light image illuminated in white, a narrow band image illuminated with narrow-band light, or a fluorescent image illuminated with excitation light.
(11) The image processing apparatus according to (10), in which
   in a case where two or more kinds of input images are input, the enhancement processing unit controls, on the basis of one kind of input image, enhancement processing on another kind of input image.
(12) An image processing method, including:
   reading an input image;
   performing enhancement processing on low-frequency components that are a range lower than a predetermined spatial frequency in an input image;
   performing enhancement processing on high-frequency components that are a spatial frequency range higher than the low-frequency components in the input image; and
   outputting an input image having the low-frequency components and the high-frequency components each subjected to enhancement processing.
(13) An endoscope system, including:
   an endoscope apparatus including an endoscope provided with an objective lens at a distal end of an insertion portion to be inserted into a body cavity, and an imaging unit that captures an optical image formed by the objective lens and outputs the optical image as an image signal, the optical image being input from the endoscope; and
   an image processing apparatus, in which
   the image processing apparatus includes
      an image reading unit that reads the image signal, and
      an enhancement processing unit that performs enhancement processing on low-frequency components that are a range lower than a predetermined spatial frequency in the image signal, performs enhancement processing on high-frequency components that are a spatial frequency range higher than the low-frequency components in the image signal, and outputs the image signal having the low-frequency components and the high-frequency components each subjected to enhancement processing.

### Reference Signs List

- 10: endoscope system
- 11: endoscope apparatus
- 12, 121, 122, 123, 123': image processing apparatus
- 13: display apparatus
- 40: excessive enhancement suppression processing unit
- 50: enhancement processing unit
- 51: low-frequency enhancement processing unit
- 52: high-frequency enhancement processing unit
- 53: image reduction unit
- 54: smoothing unit
- 55: image enlargement unit
- 56: gain multiplication unit
- 58: difference processing unit
- 59: composition processing unit
- 60: luminance/chrominance separation unit
- 61: luminance/chrominance discrimination unit
- 62: gain adjustment unit

## Claims

1. An image processing apparatus, comprising
an enhancement processing unit that performs enhancement processing on low-frequency components that are a range lower than a predetermined spatial frequency in an input image, performs enhancement processing on high-frequency components that are a range higher than the low-frequency components in the input image, and outputs the input image having the low-frequency components and the high-frequency components each subjected to enhancement processing.

2. The image processing apparatus according to claim 1, wherein
the enhancement processing unit includes a low-frequency enhancement processing unit that performs enhancement processing with respect to the low-frequency components of the input image, and
the low-frequency enhancement processing unit smoothes the input image and obtains a difference image on a basis of a difference between the input image after smoothing and the input image before smoothing.

3. The image processing apparatus according to claim 2, wherein
the low-frequency enhancement processing unit performs reduction processing on resolution of the input image at a predetermined reduction rate before the input image is smoothed.

4. The image processing apparatus according to claim 3, wherein
the low-frequency enhancement processing unit increases resolution of the difference image at an increase rate corresponding to the predetermined reduction rate after the input image is smoothed.

5. The image processing apparatus according to claim 4, wherein
the low-frequency enhancement processing unit outputs a low-frequency-enhanced image obtained by multiplying an image having the resolution increased by a predetermined coefficient and combining the multiplied image with the input image.

6. The image processing apparatus according to claim 1, wherein
the enhancement processing unit combines a low-frequency-enhanced image, which is an image having the low-frequency components subjected to enhancement processing, with the input image and performs enhancement processing on the high-frequency components of the input image combined with the low-frequency-enhanced image.

7. The image processing apparatus according to claim 1, wherein
the enhancement processing unit combines a low-frequency-enhanced image, which is an image having the low-frequency components subjected to enhancement processing, and a high-frequency enhanced image, which is an image with the high-frequency components subjected to enhancement processing, with the input image.

8. The image processing apparatus according to claim 1, wherein
the low-frequency enhancement processing unit includes
a separation processing unit that separates the input image into a luminance component image and a chrominance component image, and
a gain adjustment unit that selects, from the luminance component image and the chrominance component image, pixels to be enhanced and pixels not to be enhanced, and adjusts gains to be multiplied with respect to the pixels to be enhanced and the pixels not to be enhanced.

9. The image processing apparatus according to claim 1, wherein
the input image is a medical image.

10. The image processing apparatus according to claim 1, wherein
the input image includes at least one of a white light image illuminated in white, a narrow band image illuminated with narrow-band light, or a fluorescent image illuminated with excitation light.

11. The image processing apparatus according to claim 10, wherein
in a case where two or more kinds of input images are input, the enhancement processing unit controls, on a basis of one kind of input image, enhancement processing on another kind of input image.

12. An image processing method, comprising:
reading an input image;
performing enhancement processing on low-frequency components that are a range lower than a predetermined spatial frequency in an input image;
performing enhancement processing on high-frequency components that are a spatial frequency range higher than the low-frequency components in the input image; and
outputting an input image having the low-frequency components and the high-frequency components each subjected to enhancement processing.

13. An endoscope system, comprising:
an endoscope apparatus including an endoscope provided with an objective lens at a distal end of an insertion portion to be inserted into a body cavity, and an imaging unit that captures an optical image formed by the objective lens and outputs the optical image as an image signal, the optical image being input from the endoscope; and
an image processing apparatus, wherein
the image processing apparatus includes
an image reading unit that reads the image signal, and
an enhancement processing unit that performs enhancement processing on low-frequency components that are a range lower than a predetermined spatial frequency in the image signal, performs enhancement processing on high-frequency components that are a spatial frequency range higher than the low-frequency components in the image signal, and outputs the image signal having the low-frequency components and the high-frequency components each subjected to enhancement processing.
